(19) Europäisches Patentamt / European Patent Office / Office européen des brevets

(11) **EP 3 664 782 B1**

(12) **EUROPEAN PATENT SPECIFICATION**

(45) Date of publication and mention of the grant of the patent:
**29.01.2025  Bulletin 2025/05**

(51) International Patent Classification (IPC):
*A61K 31/745* (2006.01)   *A61K 9/16* (2006.01)
*A61K 9/50* (2006.01)   *A61K 31/785* (2006.01)
*A61P 1/00* (2006.01)   *A61P 1/12* (2006.01)

(21) Application number: **18758981.7**

(22) Date of filing: **09.08.2018**

(52) Cooperative Patent Classification (CPC):
**A61K 9/1635; A61K 9/1652; A61K 9/5026;
A61K 9/5036; A61K 9/5073; A61K 31/745;
A61K 31/785; A61P 1/00; A61P 1/12**

(86) International application number:
**PCT/SE2018/050803**

(87) International publication number:
**WO 2019/032027 (14.02.2019 Gazette 2019/07)**

(54) **CHOLESTYRAMINE PELLETS, ORAL CHOLESTYRAMINE FORMULATIONS AND MEDICAL USE THEREOF**

CHOLESTYRAMINPELLETS, ORALE CHOLESTYRAMINFORMULIERUNGEN UND IHRE MEDIZINISCHE VERWENDUNG

PASTILLES DE CHOLESTYRAMINE, FORMULATIONS ORALES DE CHOLESTYRAMINE ET LEUR UTILISATION MÉDICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **09.08.2017   SE 1750977**

(43) Date of publication of application:
**17.06.2020   Bulletin 2020/25**

(73) Proprietor: **Albireo AB**
**413 46 Göteborg (SE)**

(72) Inventors:
• **GILLBERG, Per-Göran**
**431 69 Mölndal (SE)**
• **GUSTAFSSON, Nils Ove**
**246 50 Löddeköpinge (SE)**
• **LÖVGREN, Kurt**
**435 44 Mölnlycke (SE)**

(74) Representative: **Novitas Patent AB**
**P.O. Box 55557**
**102 04 Stockholm (SE)**

(56) References cited:
EP-A1- 1 273 307     WO-A1-2017/138876
WO-A1-2017/138877     WO-A1-2017/138878
DE-A1- 3 930 168     US-A- 5 167 965

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The invention relates to small pellets comprising cholestyramine. The pellets have a high cholestyramine content and are stable enough to be coated with one or more coating layers. The invention also relates to a multiparticulate drug delivery system comprising such pellets. The invention further relates to an oral formulation for targeted delivery of cholestyramine to the colon, comprising a plurality of cholestyramine pellets that are coated with a colon release coating. The invention also relates to this formulation for use in the treatment of bile acid malabsorption and bile acid diarrhoea.

BACKGROUND

Bile acid malabsorption is a condition characterized by an excess of bile acids in the colon, often leading to chronic diarrhoea. Bile acids are steroid acids that are synthesized and conjugated in the liver. From the liver, they are excreted through the biliary tree into the small intestine where they participate in the solubilisation and absorption of dietary lipids and fat-soluble vitamins. When they reach the ileum, bile acids are reabsorbed into the portal circulation and returned to the liver. A small proportion of the secreted bile acids is not reabsorbed in the ileum and reaches the colon. Here, bacterial action results in deconjugation and dehydroxylation of the bile acids, producing the secondary bile acids deoxycholate and lithocholate.

In the colon, bile acids (in particular the dehydroxylated bile acids chenodeoxycholate and deoxycholate) stimulate the secretion of electrolytes and water. This increases the colonic motility and shortens the colonic transit time. If present in excess, bile acids produce diarrhoea with other gastrointestinal symptoms such as bloating, urgency and faecal incontinence. There have been several recent advances in the understanding of this condition of bile salt or bile acid malabsorption, or BAM (Pattni and Walters, Br. Med. Bull. 2009, vol 92, p. 79-93; Islam and Di Baise, Pract. Gastroenterol. 2012, vol. 36(10), p. 32-44). Dependent on the cause of the failure of the distal ileum to absorb bile acids, bile acid malabsorption may be divided into Type 1, Type 2 and Type 3 BAM. Diarrhoea may also be the result of high concentrations of bile acid in the large intestine following treatment with drugs that increase the production of bile acids and/or influence the reabsorption of bile acids by the small intestine, such as treatment with ileal bile acid absorption (IBAT) inhibitors.

The current treatment of bile acid malabsorption aims at binding excess bile acids in the gastrointestinal tract, beginning in the proximal part of the small bowel, thereby reducing the secretory actions of the bile acids. For this purpose, cholestyramine is commonly used as a bile acid sequestrant. Cholestyramine (or colestyramine; CAS Number 11041-12-6) is a strongly basic anion-exchange resin that is practically insoluble in water and is not absorbed from the gastrointestinal tract. Instead, it absorbs and combines with the bile acids in the intestine to form an insoluble complex. The complex that is formed upon binding of the bile acids to the resin is excreted in the faeces. The resin thereby prevents the normal reabsorption of bile acids through the enterohepatic circulation, leading to an increased conversion of cholesterol to bile acids to replace those removed from reabsorption. This conversion lowers plasma cholesterol concentrations, mainly by lowering of the low-density lipoprotein (LDL)-cholesterol.

Cholestyramine is also used as hypolipidaemic agents in the treatment of hypercholesterolemia, type II hyperlipoproteinaemia and in type 2 diabetes mellitus. It is furthermore used for the relief of diarrhoea associated with ileal resection, Crohn's disease, vagotomy, diabetic vagal neuropathy and radiation, as well as for the treatment of pruritus in patients with cholestasis.

In the current treatment of hyperlipidaemias and diarrhoea, the oral cholestyramine dose is 12 to 24 g daily, administered as a single dose or in up to 4 divided doses. In the treatment of pruritus, doses of 4 to 8 g are usually sufficient. Cholestyramine may be introduced gradually over 3 to 4 weeks to minimize the gastrointestinal effects. The most common side-effect is constipation, while other gastrointestinal side-effects are bloating, abdominal discomfort and pain, heartburn, flatulence and nausea/vomiting. There is an increased risk for gallstones due to increased cholesterol concentration in bile. High doses may cause steatorrhoea by interference with the gastrointestinal absorption of fats and concomitant decreased absorption of fat-soluble vitamins. Chronic administration may result in an increased bleeding tendency due to hypoprothrombinaemia associated with vitamin K deficiency or may lead to osteoporosis due to impaired calcium and vitamin D absorption. There are also occasional reports of skin rashes and pruritus of the tongue, skin and perianal region. Due to poor taste and texture and the various side effects, >50% of patients discontinue therapy within 12 months.

Another drawback with the current treatment using cholestyramine is that this agent reduces the absorption of other drugs administered concomitantly, such as oestrogens, thiazide diuretics, digoxin and related alkaloids, loperamide, phenylbutazone, barbiturates, thyroid hormones, warfarin and some antibiotics. It is therefore recommended that other drugs should be taken at least 1 hour before or 4 to 6 hours after the administration of cholestyramine. Dose adjustments of concomitantly taken drugs may still be necessary to perform.

In view of these side effects, it would be desirable if cholestyramine could be formulated as a colon release formulation, i.e. for release of the cholestyramine in the proximal part of the colon. Such a formulation may require a lower dose of cholestyramine and should have better properties regarding texture and taste, and may therefore be better tolerated by the patients. More importantly, colonic release of cholestyramine should be devoid of producing interactions with other drugs and should not induce risks for malabsorption of fat and fat-soluble vitamins, while still binding bile acids in order to reduce the increased colonic secretion and motility. For reasons of patient compliance, it would furthermore be desirable if the number of pills to be taken could be kept as low as possible. Each pill should therefore contain as much cholestyramine as possible.

EP 1273307 discloses preparations for preventing bile acid diarrhoea, comprising a bile acid adsorbent coated with a polymer so as to allow the release of the bile acid adsorbent around an area from the lower part of the small intestine to the cecum. It is shown that cholestyramine granules coated with HPMCAS-HF or ethyl cellulose displayed extensive swelling and bursting under conditions simulating the gastric environment.

Jacobsen et al. (Br. Med. J. 1985, vol. 290, p. 1315-1318) describe a study wherein patients who had undergone ileal resection were administered 500 mg cholestyramine tablets coated with cellulose acetate phthalate (12 tablets daily). In five of the 14 patients in this study, the tablets did not disintegrate in the desired place.

WO 2017/138876 discloses cholestyramine pellets comprising at least 70% cholestyramine. These pellets contain lower amounts of a vinylpyrrolidone-based polymer as the binding agent.

WO 2017/138877 and WO 2017/138878 disclose oral formulations for targeted delivery of cholestyramine to the colon, which formulations comprise a plurality of coated cholestyramine pellets.

US 5 167 965 A discloses extruded granules comprising cholestyramine and cellulose ether for oral delivery.

Despite progress made in this area, there still is a need for further improved cholestyramine formulations. In particular, there is a need for small cholestyramine particles that have a high cholestyramine content and are stable during the coating process.

## SUMMARY OF THE INVENTION

The invention provides small and stable pellets that have a cholestyramine content of at least 70% and that are stable enough to withstand the conditions conventionally used for applying one or more coating layers. According to claim 1, the invention discloses a population of extruded and spheronized pellets, each extruded and spheronized pellet comprising at least 70% w/w cholestyramine and

> i. a combination of at least 6% w/w of a cellulose ether and at least 2% w/w of an acrylate copolymer; or
> ii. a combination of at least 5% w/w of a cellulose ether and at least 3% w/w of an acrylate copolymer; or
> iii. a combination of at least 6% w/w of a cellulose ether, at least 1% w/w of an acrylate copolymer and at least 10% w/w microcrystalline cellulose; or
> iv. a combination of at least 5% w/w of a cellulose ether, at least 2% w/w of

an acrylate copolymer and at least 20% w/w microcrystalline cellulose.

The pellets can be coated with one or more coating layers that prevent release of the cholestyramine until the pellets reach the colon.

According to claim 7, the invention discloses a multiparticulate drug delivery system comprising a plurality of cholestyramine pellets as described herein, more particularly a drug delivery system wherein the cholestyramine pellets are formulated for colon targeted delivery.

According to claim 10, the invention also discloses an oral formulation for targeted delivery of cholestyramine to the colon, comprising a plurality of pellets as described herein and a colon release coating around said pellets. The combination of small cholestyramine pellets and a colon release coating allows the dose of cholestyramine to be reduced to for example 1.5 g twice daily. It is believed that this dose of cholestyramine is sufficient for binding an excess of bile acids in the colon. The formulation is therefore for use in the treatment or prevention of bile acid malabsorption and bile acid diarrhoea, according to claims 15 and 17.

## DETAILED DESCRIPTION OF THE INVENTION

It has been discovered that small and stable particles of cholestyramine can be obtained by extruding and spheronizing pellets from a mixture comprising cholestyramine and an appropriate binding agent. Such pellets have a high cholestyramine content and are stable enough to withstand the conditions conventionally used for applying one or more coating layers.

According to claim 1, the invention discloses a population of extruded and spheronized pellets, each extruded and spheronized pellet comprising at least 70% w/w cholestyramine and

> i. a combination of at least 6% w/w of a cellulose ether and at least 2% w/w of an acrylate copolymer; or
> ii. a combination of at least 5% w/w of a cellulose ether and at least 3% w/w of an acrylate copolymer; or
> iii. a combination of at least 6% w/w of a cellulose ether, at least 1% w/w of an acrylate copolymer and at least 10% w/w microcrystalline cellulose; or

iv. a combination of at least 5% w/w of a cellulose ether, at least 2% w/w of an acrylate copolymer and at least 20% w/w microcrystalline cellulose.

As used herein, the term "pellets" refers to extruded pellets, i.e. pellets obtained through extrusion and spheronization. The preparation of extruded pellets typically comprises the steps of mixing a powder with a liquid to obtain a wet mass, extruding the wet mass, spheronizing the extrudate and drying of the wet pellets.

It is essential that the pellets are stable enough to withstand mechanical stress during handling, such as during drying and coating of the pellets. The stability of the pellets may be expressed in terms of friability, which is the ability of a solid substance (such as a tablet, granule, sphere or pellet) to be reduced to smaller pieces, e.g. by abrasion, breakage or deformation. A low degree of friability means that the solid substance breaks into smaller pieces only to a low extent. As used herein, friability is defined as the reduction in the mass of the pellets occurring when the pellets are subjected to mechanical strain, such as tumbling, vibration, fluidization, etc. Methods for measuring friability are known in the art (e.g., European Pharmacopoeia 8.0, tests 2.9.7 or 2.9.41).

The inclusion of smaller amounts of binding agent and acrylate copolymer than specified above results in lower yield and higher friability of the pellets. Although it is not possible to define acceptable friability limits for pellets in general, friability values of <1.7% w/w friability have been reported as acceptable to withstand stresses associated with fluid bed coating, handling and other processes (Vertommen and Kinget, Drug Dev. Ind. Pharm. 1997, vol. 23, p. 39-46). For the cholestyramine pellets of the present invention, however, it has been found that a friability of 3.2% is still acceptable. The friability is preferably less than 3.5%, such as less than 3.0%, or such as less than 2.5%, or such as less than 2.0%, and more preferably less than 1.5%, even more preferably less than 1.0%, and yet even more preferably less than 0.5%.

The cellulose ether may be any cellulose ether that is suitable for pharmaceutical and oral use. Examples of suitable cellulose ethers include methyl cellulose; ethyl cellulose; ethyl methyl cellulose; ethyl hydroxyethyl cellulose (ethulose); hydroxyethyl cellulose; hydroxyethyl methyl cellulose; hydroxypropyl cellulose (HPC); hydroxypropyl methylcellulose (HPMC or hypromellose); carboxymethyl cellulose (CMC) or the sodium salt thereof (NaCMC); and mixtures comprising two or more of the aforementioned cellulose ethers.

A vinylpyrrolidone-based polymer may be polyvinylpyrrolidone (povidone) or a vinylpyrrolidone-vinyl acetate copolymer (copovidone). Povidone is a linear, water-soluble polymer made from N-vinylpyrrolidone. Copovidone (also known as copolyvidone) is a linear, water-soluble copolymer of 1-vinyl-2-pyrrolidone (povidone) and vinyl acetate in a ratio of 6:4 by mass. In a preferred embodiment, the vinylpyrrolidone-based polymer is copovidone.

The binding agent is a cellulose ether. The cellulose ether is preferably methyl cellulose, hydroxypropylcellulose, hydroxypropyl methylcellulose or sodium carboxymethyl cellulose, or a mixture comprising two or more of these cellulose ethers.

The acrylate copolymer may be any pharmaceutically acceptable copolymer comprising acrylate monomers. Examples of acrylate monomers include, but are not limited to, acrylate (acrylic acid), methyl acrylate, ethyl acrylate, methacrylic acid (methacrylate), methyl methacrylate, butyl methacrylate, trimethylammonioethyl methacrylate and dimethylaminoethyl methacrylate. Several acrylate copolymers are known under the trade name Eudragit®.

Poly(ethyl acrylate-co-methyl methacrylate-co-trimethylammonioethyl methacrylate chloride) is a copolymer of ethyl acrylate, methyl methacrylate and a low content of trimethylammonioethyl methacrylate chloride (a methacrylic acid ester with quaternary ammonium groups). The copolymer is also referred to as ammonio methacrylate copolymer. It is insoluble but the presence of the ammonium salts groups makes the copolymer permeable. The copolymer is available as a 1:2:0.2 mixture (Type A) or as a 1:2:0.1 mixture (Type B). 30% aqueous dispersions of Type A and Type B are sold under the trade names Eudragit® RL 30 D and Eudragit® RS 30 D, respectively.

Poly(methyl acrylate-co-methyl methacrylate-co-methacrylic acid) 7:3:1 is a copolymer of methyl acrylate, methyl methacrylate and methacrylic acid. It is insoluble in acidic media but dissolves by salt formation above pH 7.0. A 30% aqueous dispersion is sold under the trade name Eudragit® FS 30 D.

Poly(methacrylic acid-co-ethyl acrylate) 1:1 is a copolymer of ethyl acrylate and methacrylic acid. It is insoluble in acidic media below a pH of 5.5 but dissolves above this pH by salt formation. A 30% aqueous dispersion is sold under the trade name Eudragit® L 30 D-55.

Further suitable acrylate copolymers include poly(ethyl acrylate-co-methyl methacrylate) 2:1, which is a water-insoluble copolymer of ethyl acrylate and methyl methacrylate. 30% aqueous dispersions are sold under the trade names Eudragit® NE 30 D and Eudragit® NM 30 D.

Preferred acrylate copolymers are ammonio methacrylate copolymer, poly(methyl acrylate-co-methyl methacrylate-co-methacrylic acid) 7:3:1, and poly(methacrylic acid-co-ethyl acrylate) 1:1. More preferably, the acrylate polymer is ammonio methacrylate copolymer, and most preferably the acrylate polymer is poly(ethyl acrylate-co-methyl methacrylate-co-trimethylammonioethyl methacrylate chloride) 1:2:0.2.

The pellets may further comprise an excipient such as microcrystalline cellulose. Microcrystalline cellulose, or MCC, is a purified, partly depolymerised cellulose with shorter, crystalline polymer chains. Its binding performance makes MCC one of the most commonly used fillers and binders in drug formulations. The embodiments comprising MCC are according to claim 1.

The size of the pellets is initially governed by the diameter of the screen used in the extrusion step. After the extrusion and spheronization steps, the pellets may be sieved to obtain a pellet fraction with a narrow size distribution. The diameter of the cholestyramine pellets is preferably from 500 $\mu$m to 3000 $\mu$m, more preferably from 750 $\mu$m to 2000 $\mu$m and even more preferably from 1000 to 1600 $\mu$m. In a most preferred embodiment, the diameter of the pellets is from 1000 to 1400 $\mu$m.

Because of its physical nature, cholestyramine powder is able to absorb large amounts of water, which results in considerable swelling of the material. In order to prepare a wet mass from dry cholestyramine, it is therefore necessary to add more water than normally would be used for preparing a wet mass from dry ingredients. It has been observed that optimal conditions for forming pellets are obtained when water is added to the mix of dry ingredients in such an amount that the ingredients can form a dough-like consistency. In one embodiment, water is added to the mix of dry ingredients in a total amount of at least 1.5 times the amount of cholestyramine (w/w), more preferably in a total amount of at least 1.75 times the amount of cholestyramine (w/w), and even more preferably in a total amount of at least 2.0 times the amount of cholestyramine (w/w). In another embodiment, water is added in a total amount of at least 1.9 times the amount of dry ingredients (w/w), more preferably in a total amount of at least 2.0 times the amount of dry ingredients (w/w), and more preferably in a total amount of at least 2.1 times the amount of dry ingredients (w/w).

The uncoated pellets rapidly disintegrate under aqueous conditions. However, they are stable enough to withstand the conditions necessary for applying one or more coating layers onto the pellets.

Since the cholestyramine pellets should bind excess bile acids in the colon, they should be formulated for colon targeted delivery. This can be achieved by coating the cholestyramine pellets with one or more layers that delay the release of the cholestyramine until the pellets have reached the colon.

Therefore, in another aspect, the invention relates to a multiparticulate drug delivery system comprising a plurality of cholestyramine pellets as described herein. In a preferred embodiment, the cholestyramine pellets are formulated for colon targeted delivery. The pellets are then coated with one or more coating layers that delay release of the cholestyramine from the pellets until the coated pellets have reached the large intestine, in particular the proximal colon. In one embodiment, the colon targeted delivery is based on an enzyme-controlled release of the pellets. In another embodiment, the colon targeted delivery is based on a pH- and diffusion-controlled release of the pellets.

Because of its very low solubility, cholestyramine is not "released" from a formulation comprising coated cholestyramine pellets in that it dissolves from the formulation and diffuses into the intestine. Instead, the cholestyramine probably stays within the gradually degrading structure of the coated pellet. Therefore, as used herein, the term "release" of the cholestyramine refers to the availability of the cholestyramine to the intestinal content in order to bind components (i.e., bile acids) therein.

In another aspect, the invention relates to an oral formulation for targeted delivery of cholestyramine to the colon, comprising

a) a plurality of pellets according to the invention; and
b) a colon release coating around said pellets.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein more than 70% of the cholestyramine is released in the colon.

In some embodiments, more than 75% of the cholestyramine is released in the colon. In other embodiments, more than 80% of the cholestyramine is released in the colon. In other embodiments, more than 85% of the cholestyramine is released in the colon. In yet other embodiments, more than 90% of the cholestyramine is released in the colon.

In yet another aspect, the invention relates to an oral formulation, comprising:

a) a plurality of pellets, according to the invention; and

b) a coating surrounding said pellets, wherein the coating is capable of targeting release of the cholestyramine in the colon,

wherein less than 30% of the cholestyramine is released in the small intestine.

In some embodiments, less than 25% of the cholestyramine is released in the small intestine. In other embodiments, less than 20% of the cholestyramine is released in the small intestine. In other embodiments, less than 15% of the cholestyramine is released in the small intestine. In yet other embodiments, less than 10% of the cholestyramine is released in the small intestine.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein the pellets exhibit a friability of less than 3.5% as measured using the European Pharmacopoeia 8.0, test 2.9.7.

In some embodiments, the pellets exhibit a friability of less than 3.0%. In other embodiments, the pellets exhibit a friability of less than 2.5%. In other embodiments, the pellets exhibit a friability of less than 2.0%. In other embodiments, the pellets exhibit a friability of less than 1.5%. In other embodiments, the pellets exhibit a friability of less than 1.0%. In yet other embodiments, the pellets exhibit a friability of less than 0.5%.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein less than 30% of the cholestyramine is released after 6 hours at pH of 5.5 as measured using the USP Dissolution Apparatus 2 (paddle) Ph. Eur. 2.9.3.

In some embodiments, less than 25% of the cholestyramine is released after 6 hours at pH of 5.5 as measured using the USP Dissolution Apparatus 2 (paddle) Ph. Eur. 2.9.3. In other embodiments, less than 20% of the cholestyramine is released after 6 hours at pH of 5.5 as measured using the USP Dissolution Apparatus 2 (paddle) Ph. Eur. 2.9.3. In other embodiments, less than 15% of the cholestyramine is released after 6 hours at pH of 5.5 as measured using the USP Dissolution Apparatus 2 (paddle) Ph. Eur. 2.9.3. In yet other embodiments, less than 10% of the cholestyramine is released after 6 hours at pH of 5.5 as measured using the USP Dissolution Apparatus 2 (paddle) Ph. Eur. 2.9.3.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein the formulation exhibits less than 30% sequestration of cholic acid after 6 hours at pH 5.5 as measured using a USP Dissolution Apparatus 2 (paddle) Ph. Eur. 2.9.3.

In some embodiments, the formulation exhibits less than 25% sequestration of cholic acid after 6 hours at pH 5.5 as measured using a USP Dissolution Apparatus 2 (paddle) Ph. Eur. 2.9.3. In other embodiments, the formulation exhibits less than 20% sequestration of cholic acid after 6 hours at pH 5.5 as measured using a USP Dissolution Apparatus 2 (paddle) Ph. Eur. 2.9.3. In yet other embodiments, the formulation exhibits less than 15% sequestration of cholic acid after 6 hours at pH 5.5 as measured using a USP Dissolution Apparatus 2 (paddle) Ph. Eur. 2.9.3.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein the formulation exhibits greater than 30% sequestration of cholic acid after 2 hours at pH 1 followed by 4 hours at pH 6.8 as measured using a USP Dissolution Apparatus 2 (paddle) Ph. Eur. 2.9.3.

In some embodiments, the formulation exhibits greater than 35% sequestration of cholic acid after 2 hours at pH 1 followed by 4 hours at pH 6.8 as measured using a USP Dissolution Apparatus 2 (paddle) Ph. Eur. 2.9.3. In other embodiments, the formulation exhibits greater than 40% sequestration of cholic acid after 2 hours at pH 1 followed by 4 hours at pH 6.8 as measured using a USP Dissolution Apparatus 2 (paddle) Ph. Eur. 2.9.3. In yet other embodiments, the formulation exhibits greater than 45% sequestration of cholic acid after 2 hours at pH 1 followed by 4 hours at pH 6.8 as measured using a USP Dissolution Apparatus 2 (paddle) Ph. Eur. 2.9.3. In yet other embodiments, the formulation exhibits greater than 50% sequestration of cholic acid after 2 hours at pH 1 followed by 4 hours at pH 6.8 as measured using a USP Dissolution Apparatus 2 (paddle) Ph. Eur. 2.9.3.

In another aspect, the invention relates to an oral formulation, comprising:

a) a plurality of pellets, according to the invention; and

b) a coating surrounding said pellets, wherein the coating is capable of targeting release of the cholestyramine in the colon,

wherein the formulation exhibits less than 30% sequestration of cholic acid after 2 hours at pH 1 as measured using a USP Dissolution Apparatus 2 (paddle) Ph. Eur. 2.9.3.

In some embodiments, the formulation exhibits less than 25% sequestration of cholic acid after 2 hours at pH 1 as measured using a USP Dissolution Apparatus 2 (paddle) Ph. Eur. 2.9.3. In other embodiments, the formulation exhibits less than 20% sequestration of cholic acid after 2 hours at pH 1 as measured using a USP Dissolution Apparatus 2 (paddle) Ph. Eur. 2.9.3. In other embodiments, the formulation exhibits less than 15% sequestration of cholic acid after 2 hours at pH 1 as measured using a USP Dissolution Apparatus 2 (paddle) Ph. Eur. 2.9.3. In yet other embodiments, the formulation exhibits less than 10% sequestration of cholic acid after 2 hours at pH 1 as measured using a USP Dissolution Apparatus 2 (paddle) Ph. Eur. 2.9.3.

In yet another aspect, the invention relates to an oral formulation, comprising:

a) a plurality of pellets, according to the invention; and

b) a coating surrounding said pellets, wherein the coating is capable of targeting release of the cholestyramine in the colon,

wherein the formulation exhibits greater than 30% sequestration of cholic acid after 2 hours at pH 1 followed by 4 hours at pH 7.4 as measured using a USP Dissolution Apparatus 2 (paddle) Ph. Eur. 2.9.3.

In some embodiments, the formulation exhibits greater than 35% sequestration of cholic acid after 2 hours at pH 1 followed by 4 hours at pH 7.4 as measured using a USP Dissolution Apparatus 2 (paddle) Ph. Eur. 2.9.3. In other embodiments, the formulation exhibits greater than 40% sequestration of cholic acid after 2 hours at pH 1 followed by 4 hours at pH 7.4 as measured using a USP Dissolution Apparatus 2 (paddle) Ph. Eur. 2.9.3. In other embodiments, the formulation exhibits greater than 45% sequestration of cholic acid after 2 hours at pH 1 followed by 4 hours at pH 7.4 as measured using a USP Dissolution Apparatus 2 (paddle) Ph. Eur. 2.9.3. In yet other embodiments, the formulation exhibits greater than 50% sequestration of cholic acid after 2 hours at pH 1 followed by 4 hours at pH 7.4 as measured using a USP Dissolution Apparatus 2 (paddle) Ph. Eur. 2.9.3.

The colon release coating should also prevent the cholestyramine pellets from bursting. When water that diffuses through the coating is absorbed by the cholestyramine, the increasing volume of the cholestyramine leads to swelling of the pellets. The coating of the pellets should for that reason be sufficiently elastic in order to withstand the swelling of the pellets. By preventing the pellets from bursting, the coating avoids premature release of the cholestyramine.

The colon release coating consists of one or more coating layers that delay the availability of the cholestyramine to the intestinal content until the pellets have reached the desired part of the colon. Techniques based on changes in the bacterial environment (i.e., enzyme-controlled release) or pH (pH-controlled release), based on gradual erosion of the coating (time-controlled release) or based on diffusion through a permeable film (diffusion-controlled release), or a combination of two or more of the above techniques may be used for controlling the release position and the rate of release of the pellets.

*Enzyme-controlled release coating*

In one embodiment, the colon release coating around the pellets allows for enzyme-controlled release of the cholestyramine in the colon. The coating layer then comprises a biodegradable polymer that is degraded by bacterial enzymes present in the colon, but that is not degraded by the human enzymes present in the gastrointestinal tract. The release of the cholestyramine from the pellets is thus triggered by changes in the bacterial environment and substantially prevented until the coated pellets reach the colon.

The biodegradable polymer may be an azo polymer or a polysaccharide. Examples of bacterially degradable polysaccharides include chitosan, pectin, guar gum, dextran, inulin, starch and amylose, as well as derivatives thereof (Sinha and Kumria, Eur. J. Pharm. Sci. 2003, vol. 18, p. 3-18). The colon release coating preferably comprises starch.

The structure of starch generally comprises 20-30% (w/w) amylose, which is less easily degraded by intestinal microbiota, and 70-80% (w/w) amylopectin, which is more easily degraded by intestinal microbiota. Thus, depending on the specific amounts of amylose and amylopectin present in the structure, different types of starch have different degradation profiles. Resistant starch has a high amylose content and generally escapes from digestion in the small intestine. Such starch is instead digested by bacteria in the colon. Depending on the natural source of the starch and how it has been treated, resistant starch can be categorized into four types (RS1 to RS4), each having different properties. Resistant starch type 2 (RS2), such as in high amylose maize starch (or high amylose corn starch) is less accessible to enzymes due to the conformation of the starch. The colon release coating around the cholestyramine pellets preferably comprises resistant starch type 2 (RS2). When RS2 is cooked or heated, realignment of the amylose and amylopectin crystalline structures occurs in a process called retrogradation, leading to resistant starch type 3 (RS3).

In addition to the biodegradable polymer, the coating layer comprises one or more further organic polymers. Examples of suitable organic polymers include, but are not limited to, poly(methyl acrylate-co-methyl methacrylate-co-methacrylic acid) 7:3:1 (Eudragit® FS 30 D), poly(ethyl acrylate-co-methyl methacrylate-co-trimethylammonioethyl methacrylate chloride) 1:2:0.2 (Eudragit® RL 30 D), poly(ethyl acrylate-co-methyl methacrylate-co-trimethylammonioethyl methacrylate chloride) 1:2:0.1 (Eudragit® RS 30 D), poly(ethyl acrylate-co-methyl methacrylate) 2:1 (Eudragit® NE 30 D or Eudragit® NM 30 D) and poly(vinyl acetate) (e.g., Kollicoat® SR 30 D). Preferably, the organic polymer is poly(methyl acrylate-co-methyl methacrylate-co-methacrylic acid) 7:3:1 (Eudragit® FS 30 D).

*pH- and diffusion-controlled coating*

In another embodiment, the colon release coating around the pellets allows for pH- and diffusion-controlled release of the cholestyramine in the colon. The coating then comprises a diffusion-controlled inner coating layer around the pellets and an enteric (pH-controlled) outer coating layer.

The diffusion-controlled inner coating layer provides a modified release of the cholestyramine, i.e. the cholestyramine is not made available at once but over an extended period of time. The coating layer comprises one or more polymers that are insoluble at any pH value, but that are permeable to water and small molecules dissolved therein. Examples of such polymers include, but are not limited to, poly(ethyl acrylate-co-methyl methacrylate-co-trimethylammonioethyl methacrylate chloride) 1:2:0.2 (Eudragit® RL 30 D), poly(ethyl acrylate-co-methyl methacrylate-co-trimethylammonioethyl methacrylate chloride) 1:2:0.1 (Eudragit® RS 30 D), poly(ethyl acrylate-co-methyl methacrylate) 2:1 (Eudragit® NE 30 D or Eudragit® NM 30 D) and polyvinyl acetate (Kollicoat® SR 30 D). The diffusion-controlled inner coating preferably comprises poly(ethyl acrylate-co-methyl methacrylate-co-trimethylammonioethyl methacrylate chloride) 1:2:0.2 (Eudragit® RL 30 D), poly(ethyl acrylate-co-methyl methacrylate-co-trimethylammonioethyl methacrylate chloride) 1:2:0.1 (Eudragit® RS 30 D) or a combination thereof, and most preferably poly(ethyl acrylate-co-methyl methacrylate-co-trimethylammonioethyl methacrylate chloride) 1:2:0.1.

The enteric coating layer comprises a pH-sensitive polymer that is stable and insoluble at the acidic pH values found in the stomach (pH ~1-3) but that breaks down rapidly or becomes soluble at less acidic pH values, such as the pH values found in the small intestine (pH ~6 to 7). Examples of such pH-sensitive polymers include, but are not limited to, cellulose acetate phthalate, cellulose acetate succinate, hydroxypropyl methylcellulose acetate succinate, hydroxypropyl methylcellulose phthalate, poly(methacrylic acid-co-methyl methacrylate) 1:1 (Eudragit® L 100), poly(methacrylic acid-co-methyl methacrylate) 1:2 (Eudragit® S 100), poly(methacrylic acid-co-ethyl acrylate) 1:1 (Eudragit® L 100-55), poly(methyl acrylate-co-methyl methacrylate-co-methacrylic acid) 7:3:1 (Eudragit® FS 30 D), polyvinyl acetate phthalate, shellac, sodium alginate, and zein, as well as mixtures thereof. The enteric coating preferably comprises a pH-sensitive polymer selected from the group consisting of poly(methacrylic acid-co-methyl methacrylate) 1:1, hydroxypropyl methylcellulose acetate succinate and poly(methacrylic acid-co-methyl methacrylate) 1:2. The enteric coating most preferably comprises hydroxypropyl methylcellulose acetate succinate.

When water is absorbed by the cholestyramine, the increasing volume of the cholestyramine leads to swelling of the pellets. The enzyme-controlled coating layer or the diffusion-controlled inner coating layer should therefore be elastic (i.e., have high elongation at break). Because of the elasticity of the coating layers, the coating is able to withstand this swelling. Burst of the pellets and premature release of the cholestyramine is thereby avoided. The elasticity of the coating may be the result of the elasticity of the organic polymer(s) itself, or may be induced by the addition of a plasticizer.

Examples of suitable plasticizers include, but are not limited to, triethyl citrate, glyceryl triacetate, tributyl citrate, diethyl phthalate, acetyl tributyl citrate, dibutyl phthalate and dibutyl sebacate.

In order to improve the adherence of the coating layer onto the cholestyramine pellets, or in order to minimize the interaction between the coating layer(s) and the cholestyramine in the pellets, an additional barrier coating layer may optionally be present between the pellet and the coating layer. A barrier coating layer may also be present when two different coating layers should be kept physically separated from each other. A particularly suitable material for the barrier coating layer is hydroxypropyl methylcellulose (HPMC).

The controlled release coating layer(s) and the optional barrier coating layer(s) may comprise one or more additives, such as acids and bases, plasticizers, glidants, and surfactants. Examples of suitable acids include organic acids such as citric acid, acetic acid, trifluoroacetic acid, propionic acid, succinic acid, glycolic acid, lactic acid, malic acid, tartaric acid, ascorbic acid, pamoic acid, maleic acid, hydroxymaleic acid, phenylacetic acid, glutamic acid, benzoic acid, salicylic acid, mesylic acid, esylic acid, besylic acid, sulfanilic acid, 2-acetoxybenzoic acid, fumaric acid, toluenesulfonic acid, methanesulfonic acid, ethane disulfonic acid and oxalic acid, and inorganic acids such as hydrochloric acid, hydrobromic acid, sulphuric acid, sulfamic acid, phosphoric acid and nitric acid. Examples of suitable bases include inorganic bases such as sodium bicarbonate, sodium hydroxide and ammonium hydroxide. Examples of suitable plasticizers include triethyl citrate, glyceryl triacetate, tributyl citrate, diethyl phthalate, acetyl tributyl citrate, dibutyl phthalate and dibutyl sebacate. Examples of suitable glidants include talc, glyceryl monostearate, oleic acid, medium chain triglycerides and colloidal silicon dioxide. Examples of suitable surfactants include sodium dodecyl sulfate, polysorbate 80 and sorbitan monooleate.

A thin layer of a non-sticking agent may ultimately be applied to the coated pellets. This outer layer prevents the coated pellets from sticking together, e.g. during storage. Examples of suitable non-sticking agents include fumed silica, talc and magnesium stearate.

The coating layers may be applied onto the cholestyramine pellets by methods known in the art, such as by film coating involving perforated pans and fluidized beds.

The colon release coating substantially prevents release of the cholestyramine from the pellets until they have reached the large intestine. Preferably, there should be no exposure of the cholestyramine in the small intestine, whereas the exposure should be quick once the multiparticulates have passed the ileocecal valve. In one embodiment, less than 30% of the cholestyramine is released in the small intestine, such as less than 20%, such as less than 10%. In a more preferred embodiment, less than 5% of the cholestyramine is released in the small intestine. In another embodiment, more than 70% of the cholestyramine is released in the colon, such as more than 80%, such as more than 90%. In a more preferred embodiment, more than 95% of the cholestyramine is released in the colon.

The colon release coating adds further weight and volume to the pellets. The smaller the size of the pellets, the larger is the impact of the coating on the volume of the final formulation. However, for reasons of patient compliance, it is desirable that the total volume of the formulation is kept as low as possible. The coating layer(s) should therefore be as thin as possible. Preferably, the amount of coating in the final formulation (on dry weight basis) is less than 50% w/w, more preferably less than 45% w/w, more preferably less than 40% w/w and even more preferably less than 35% w/w.

The cholestyramine content of the pellets should be as high as possible. The uncoated pellets therefore preferably contain at least 75% w/w cholestyramine, more preferably at least 80% w/w cholestyramine, even more preferably at least 85% w/w cholestyramine and most preferably at least 90% w/w cholestyramine.

The oral formulation described herein may be administered to a patient in different forms, depending on factors such as the age and general physical condition of the patient. For example, the formulation may be administered in the form of one or more capsules wherein the coated pellets are contained. Such capsules conventionally comprise a degradable material, such as gelatin, hydroxypropyl methylcellulose (HPMC), pullulan or starch, which easily disintegrates under the acidic conditions in the stomach. The coated pellets are thereby quickly released into the stomach. Thus, in one aspect, the invention relates to a capsule comprising the oral formulation disclosed herein.

Alternatively, the coated pellets may be administered as a sprinkle formulation, the contents of which can be dispersed in liquid or soft food. Such a formulation does not require the swallowing of larger capsules and is therefore particularly useful for infants and small children as well as for older adults. Thus, in another aspect, the invention relates to a sprinkle formulation comprising the oral formulation disclosed herein. In such a formulation, the coated pellets may be contained within a capsule, sachet or stick pack.

The oral formulation disclosed herein provides several advantages over other formulations. The small coated pellets (multiparticulates) according to the present invention are able to easily pass the gastrointestinal tract. This eliminates the risk that the formulation is temporarily held up in the gastrointestinal tract, such as at the stomach or at the ileocecal valve, as is sometimes encountered with monolithic formulations (such as tablets or capsules that do not disintegrate in the stomach). Furthermore, the cholestyramine is made available to the intestinal content only when the colon release coating starts being degraded in the lower gastrointestinal tract, in particular the colon. The contents of the stomach and the small intestine are therefore effectively protected from the cholestyramine, which is a major improvement over formulations that directly release the cholestyramine in the stomach or the small intestine. Because the cholestyramine is made available to the intestinal content only after reaching the colon, the oral formulation disclosed herein also reduces undesired interactions of cholestyramine with other components in the gastrointestinal tract, such as other drugs or nutrients.

The low solubility of cholestyramine in aqueous environment prevents the release of cholestyramine from the formulation to be measured directly. The availability of the cholestyramine to the intestinal content over time and at different pH values can instead be determined *in vitro,* such as by measuring the sequestering capacity of the formulation under simulated conditions for the gastrointestinal tract. Such a method involves measuring the decreasing amount of free bile acid (i.e., the compound to be sequestered) in a liquid medium representative of the gastrointestinal tract, as described in the experimental section. See also the Official Monograph for cholestyramine resin (USP 40, page 3404).

For instance, the sequestering capacities of the cholestyramine formulations may be studied using the Simulator of the Human Intestinal Microbial Ecosystem (SHIME®) as developed by ProDigest (Ghent, Belgium). As described in more detail in the experimental section, this model enables the *in vitro* evaluation of the bile acid binding capacity of cholestyramine formulations under physiological conditions representative for fasted stomach, small intestine and proximal colon. Bile acids such as cholic acid (CA), chenodeoxycholic acid (CDCA) and deoxycholic acid (DCA) may be used in such studies, or a mixture of two or more of these bile salts. A 40:40:20 (w/w) mixture of CA, CDCA and DCA is preferably used as a representative mixture of human bile salts. Experiments on cholestyramine formulations should be run in parallel with a control experiment to which no cholestyramine is added, in order to monitor the degradation of the bile salts under the conditions used in the assay. For each experiment, samples are taken at selected time intervals and the concentrations of the bile acids in the samples are determined, e.g. by means of HPLC. From these data, the percentage of remaining bile acids in each studied sample may be calculated as the ratio of the value of the studied sample to the value of the control sample at the corresponding incubation time:

$$\% \ remaining \ bile \ acid = \frac{concentration \ of \ BA \ in \ sample}{concentration \ of \ BA \ in \ control \ sample} \times 100$$

A plot of the percentage of remaining bile acids against time will show the decrease of bile acids, i.e. the sequestration of bile acids by the cholestyramine formulations, during small intestinal and colonic incubation.

In another aspect, the invention relates to an oral formulation, comprising:

a) a plurality of pellets, according to the invention; and
b) a coating surrounding each pellet, wherein the coating is capable of targeting release of the cholestyramine in the colon;

wherein the oral formulation herein exhibits less than about 30% sequestration of one or more of cholic acid, chenodeoxycholic acid, and deoxycholic acid after 2 hours in small intestinal incubations as measured in the Simulator of the Human Intestinal Microbial Ecosystem (SHIME) model.

In some embodiments, the oral formulation exhibits less than about 25% sequestration of one or more of cholic acid, chenodeoxycholic acid, and deoxycholic acid after 2 hours in small intestinal incubations as measured in the Simulator of the Human Intestinal Microbial Ecosystem (SHIME) model. In other embodiments, the oral formulation exhibits less than about 20% sequestration of one or more of cholic acid, chenodeoxycholic acid, and deoxycholic acid after 2 hours in small intestinal incubations as measured in the Simulator of the Human Intestinal Microbial Ecosystem (SHIME) model. In yet other embodiments, the oral formulation exhibits less than about 15% sequestration of one or more of cholic acid, chenodeoxycholic acid, and deoxycholic acid after 2 hours in small intestinal incubations as measured in the Simulator of the Human Intestinal Microbial Ecosystem (SHIME) model.

In another aspect, the invention relates to the formulation disclosed herein for use in the treatment or prevention of bile acid malabsorption.

Herein is also described the use of the formulation according to the invention in the manufacture of a medicament for the treatment or prevention of bile acid malabsorption.

Herein is also described the formulation according to the invention for use in the treatment or prevention of bile acid malabsorption comprising administering to a mammal in need of such treatment or prevention a therapeutically effective amount of the formulation disclosed herein.

Bile acid malabsorption may be divided into three different types, dependent on the cause of the failure of the distal ileum to absorb bile acids. Type 1 BAM is the result of (terminal) ileal disease (such as Crohn's disease) or (terminal) ileal resection or bypass. Type 2 BAM is often referred to as idiopathic bile acid malabsorption or primary bile acid diarrhoea (BAD) and is believed to be the result of an overproduction of bile acids or caused by a defective feedback inhibition of hepatic bile acid synthesis. This feedback regulation is mediated by the ileal hormone fibroblast growth factor 19 (FGF19) in man. Finally, type 3 BAM may be the result of cholecystectomy, vagotomy, small intestinal bacterial overgrowth (SIBO), coeliac disease, pancreatic insufficiency (chronic pancreatitis, cystic fibrosis), pancreatic transplant, radiation enteritis, collagenous colitis, microscopic colitis, lymphocytic colitis, ulcerative colitis or irritable bowel syndrome (i.e., diarrhoea-predominant irritable bowel syndrome (IBS-D)).

The formulation may also be used in combination with an Ileal Bile Acid Absorption (IBAT) inhibitor. Treatment with IBAT inhibitors, such as in the treatment of liver diseases, disorders of fatty acid metabolism or glucose utilization disorders, may result in increased levels of bile acids and/or influence the reabsorption of bile acids by the small intestine, leading to high concentrations of bile acid in the large intestine and thus causing diarrhoea. This side effect of the treatment with IBAT inhibitors may be treated or prevented by treatment with the formulation as disclosed herein. The formulation and the IBAT inhibitor may be administered simultaneously, sequentially or separately. Thus, in another aspect, the invention relates to the formulation disclosed herein, for use in the treatment or prevention of diarrhoea upon oral administration of an IBAT inhibitor.

Herein is also described the use of the formulation according to the invention in the manufacture of a medicament for the treatment or prevention of diarrhoea upon oral administration of an IBAT inhibitor. Herein is also described the formulation of the invention for use in the treatment or prevention of diarrhoea upon oral administration of an IBAT inhibitor, comprising administering to a mammal in need of such treatment or prevention therapeutically effective amounts of an IBAT inhibitor and of the formulation disclosed herein.

In a preferred embodiment, the invention relates to the formulation disclosed herein, for use in the treatment or prevention of bile acid diarrhoea upon treatment of a liver disease, such as a cholestatic liver disease, comprising oral administration of an IBAT inhibitor. In particular, the invention relates to the formulation disclosed herein for use in the treatment or prevention of diarrhoea upon treatment of Alagilles syndrome (ALGS), progressive familial intrahepatic cholestasis (PFIC), primary biliary cirrhosis (PBC), primary sclerosing cholangitis (PSC), autoimmune hepatitis, cholestatic pruritus, non-alcoholic fatty liver disease (NAFLD) or non-alcoholic steatohepatitis (NASH) comprising oral administration of an IBAT inhibitor.

Herein is also described the formulation of the invention for use in the treatment or prevention of bile acid diarrhoea upon treatment of a liver disease comprising oral administration of an IBAT inhibitor, comprising administering to a mammal in need of such treatment or prevention a therapeutically effective amount of the formulation disclosed herein. In particular, the invention relates to such a method for the treatment or prevention of diarrhoea wherein the liver disease is Alagilles syndrome (ALGS), progressive familial intrahepatic cholestasis (PFIC), primary biliary cirrhosis (PBC), primary sclerosing cholangitis (PSC), autoimmune hepatitis, cholestatic pruritus, non-alcoholic fatty liver disease (NAFLD) or non-alcoholic steatohepatitis (NASH).

A liver disease as defined herein is any bile acid-dependent disease in the liver and in organs connected therewith, such as the pancreas, portal vein, the liver parenchyma, the intrahepatic biliary tree, the extrahepatic biliary tree, and the gall bladder. Liver diseases include, but are not limited to an inherited metabolic disorder of the liver; inborn errors of bile acid synthesis; congenital bile duct anomalies; biliary atresia; neonatal hepatitis; neonatal cholestasis; hereditary forms of cholestasis; cerebrotendinous xanthomatosis; a secondary defect of BA synthesis; Zellweger's syndrome; cystic fibrosis (manifestations in the liver); alpha1-antitrypsin deficiency; Alagilles syndrome (ALGS); Byler syndrome; a primary defect of bile acid (BA) synthesis; progressive familial intrahepatic cholestasis (PFIC) including PFIC-1, PFIC-2, PFIC-3 and non-specified PFIC; benign recurrent intrahepatic cholestasis (BRIC) including BRIC1, BRIC2 and non-specified BRIC; autoimmune hepatitis; primary biliary cirrhosis (PBC); liver fibrosis; non-alcoholic fatty liver disease (NAFLD); non-alcoholic steatohepatitis (NASH); portal hypertension; general cholestasis; jaundice during pregnancy; jaundice due to drugs; intrahepatic cholestasis; extrahepatic cholestasis; primary sclerosing cholangitis (PSC); gall stones and choledocholithiasis; malignancy causing obstruction of the biliary tree; pruritus due to cholestasis or jaundice; pancreatitis; chronic autoimmune liver disease leading to progressive cholestasis; hepatic steatosis; alcoholic hepatitis; acute fatty liver; fatty liver of pregnancy; drug-induced hepatitis; iron overload disorders; hepatic fibrosis; hepatic cirrhosis; amyloidosis; viral hepatitis; and problems in relation to cholestasis due to tumours and neoplasms of the liver, of the biliary tract and of the pancreas.

Disorders of fatty acid metabolism and glucose utilization disorders include, but are not limited to, hypercholesterolemia, dyslipidemia, metabolic syndrome, obesity, disorders of fatty acid metabolism, glucose utilization disorders, disorders in which insulin resistance is involved, and type 1 and type 2 diabetes mellitus.

IBAT inhibitors are often referred to by different names. As used herein, the term "IBAT inhibitors" should be understood as also encompassing compounds known in the literature as Apical Sodium-dependent Bile Acid Transporter Inhibitors (ASBTI's), bile acid transporter (BAT) inhibitors, ileal sodium/bile acid cotransporter system inhibitors, apical sodium-bile acid cotransporter inhibitors, ileal sodium-dependent bile acid transport inhibitors, bile acid reabsorption inhibitors (BARI's), and sodium bile acid transporter (SBAT) inhibitors.

IBAT inhibitors that can be used in combination with the bile acid sequestrant formulation disclosed herein include, but are not limited to, benzothiazepines, benzothiepines, 1,4-benzothiazepines, 1,5-benzothiazepines and 1,2,5-benzothiadiazepines.

Suitable examples of IBAT inhibitors that can be used in combination with the bile acid sequestrant formulation disclosed herein include, but are not limited to, the compounds disclosed in WO 93/16055, WO 94/18183, WO 94/18184, WO 96/05188, WO 96/08484, WO 96/16051, WO 97/33882, WO 98/03818, WO 98/07449, WO 98/40375, WO 99/35135, WO 99/64409, WO 99/64410, WO 00/47568, WO00/61568, WO 00/38725, WO 00/38726, WO 00/38727, WO 00/38728, WO 00/38729, WO 01/68096, WO 02/32428, WO 03/061663, WO 2004/006899, WO 2007/009655, WO 2007/009656, DE 19825804, EP 864582, EP 489423, EP 549967, EP 573848, EP 624593, EP 624594, EP 624595, EP 624596, EP 0864582, EP 1173205 and EP 1535913.

Particularly suitable IBAT inhibitors are those disclosed in WO 01/66533, WO 02/50051, WO 03/022286, WO 03/020710, WO 03/022825, WO 03/022830, WO 03/091232, WO 03/106482 and WO 2004/076430, and especially the compounds selected from the group consisting of:

1,1-dioxo-3,3-dibutyl-5-phenyl-7-methylthio-8-(N-{(R)-α-[N-(carboxymethyl)carbamoyl]-benzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N'-((S)-1-carboxyethyl)carbamoyl-benzyl}carbamoylmethoxy)-2,3,4,5-tetrahydro-1,5-benzothiazepine;

1,1-dioxo-3,3-dibutyl-5-phenyl-7-methylthio-8-(N-{(R)-α-[N-((S)-1-carboxypropyl)-carbamoyl]benzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((R)-1-carboxy-2-methylthioethyl)-carbamoyl]benzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxypropyl)carbamoyl]-4-hydroxybenzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((R)-1-carboxy-2-methylthioethyl)carbamoyl]-4-hydroxybenzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxy-2-methylpropyl)-carbamoyl]benzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxy-2-(R)-hydroxypropyl)carbamoyl]-4-hydroxybenzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxybutyl)carbamoyl]-4-hydroxybenzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxyethyl)carbamoyl]-benzyl}carbamoylmethoxy)-2,3,4,5-tetrahydro-1,2,5-benzothiadiazepine;

1, 1-dioxo-3, 3-dibutyl-5-phenyl-7-methylthio-8- (N-{(R)-α-[N'-((S)-1-carboxypropyl)carbamoyl]-4-hydroxybenzyl} carbamoylmethoxy)-2,3,4,5-tetrahydro-1,5-benzothiazepine;

1,1-dioxo-3,3-dibutyl-5-phenyl-7-methylthio-8-(N-{(R)-α-[N-((S)-1-carboxyethyl)carbamoyl]-4-hydroxybenzyl}carbamoylmethoxy)-2,3,4,5-tetrahydro-1,2,5-benzothiadiazepine;

1,1-dioxo-3,3-dibutyl-5-phenyl-7-methylthio-8-(N-{(R)-α-[N-((S)-1-carboxy-2-methylpropyl)-carbamoyl]-4-hydroxybenzyl}carbamoylmethoxy)-2,3,4,5-tetrahydro-1,2,5-benzothiadiazepine; and

1,1-dioxo-3,3-dibutyl-5-phenyl-7-methylthio-8-(N-{(R)-1'-phenyl-1'-[N'-(carboxymethyl)carbamoyl] methyl}carbamoylmethoxy)-2,3,4,5-tetrahydro-1,5-benzothiazepine;

or a pharmaceutically acceptable salt thereof.

Other particularly suitable IBAT inhibitors are those disclosed in WO99/32478, WO00/01687, WO01/68637, WO03/022804, WO 2008/058628 and WO 2008/058630, and especially the compounds selected from the group consisting of:

1-[4-[4-[(4R,5R)-3,3-dibutyl-7-(dimethylamino)-2,3,4,5-tetrahydro-4-hydroxy-1,1-dioxido-1-benzothiepin-5-yl]phenoxy]butyl]4-aza-1-azoniabicyclo[2.2.2]octane methanesulfonate;

1-[[4-[[4-[3,3-dibutyl-7-(dimethylamino)-2,3,4,5-tetrahydro-4-hydroxy-1,1-dioxido-1-benzothiepin-5-yl]phenoxy] methyl]phenyl]methyl]-4-aza-1-azoniazabicyclo[2.2.2]octane chloride;

1-[[5-[[3-[(3S,4R,5R)-3-butyl-7-(dimethylamino)-3-ethyl-2,3,4,5-tetrahydro-4-hydroxy-1,1-dioxido-1-benzothiepin-5-yl]phenyl]amino]-5-oxopentyl]amino]-1-deoxy-D-glucitol; and

potassium ((2R,3R,4S,5R,6R)-4-benzyloxy-6-{3-[3-((3S,4R,5R)-3-butyl-7-dimethylamino-3-ethyl-4-hydroxy-1,1-dioxo-2,3,4,5-tetrahydro-1H-benzo[b]thiepin-5-yl)-phenyl]-ureido}-3,5-dihydroxy-tetrahydro-pyran-2-ylmethyl)sulphate, ethanolate, hydrate.

An effective amount of the cholestyramine formulation according to the invention can be any amount containing more than or equal to about 100 mg of cholestyramine, such as more than or equal to about 250 mg, 500 mg, 750 mg, 1000 mg, 1250 mg, 1500 mg, 1750 mg or 2000 mg of cholestyramine. For example, the effective amount of cholestyramine can be between 100 mg and 5000 mg, such as between 250 mg and 2500 mg, between 250 mg and 2000 mg, between 500 mg and 2500 mg, between 500 mg and 2000 mg, or between 750 mg and 2000 mg.

A unit dose of the cholestyramine formulation according to the invention may comprise from 200 to 300 mg of cholestyramine, such as from 220 to 280 mg of cholestyramine, such as from 240 to 260 mg of cholestyramine. A unit dose preferably comprises about 250 mg of cholestyramine. The daily dose can be administered as a single dose or divided into one, two, three or more unit doses.

The frequency of administration of the formulation as disclosed herein can be any frequency that reduces the bile acid malabsorption condition without causing any significant adverse effects or toxicity to the patient. The frequency of administration can vary from once or twice a week to several times a day, such as once a day or twice a day. The frequency of administration can furthermore remain constant or be variable during the duration of the treatment.

Several factors can influence the frequency of administration and the effective amount of the formulation that should be used for a particular application, such as the severity of the condition being treated, the duration of the treatment, as well as the age, weight, sex, diet and general medical condition of the patient being treated.

The invention is further illustrated by means of the following examples, which do not limit the invention in any respect.

EXAMPLES

**Example 1**

**Extrusion/spheronization experiments**

*Experiments with different amounts of binding agent (Methocel™ E3)*

All experiments were performed on a 200 g scale. The dry ingredients (cholestyramine, Methocel™ E3 (hydroxypropyl methylcellulose, viscosity ~3 mPa·s) and microcrystalline cellulose; see amounts in table 1 below) were mixed in a Kenwood Patissier for 1 minute. When Eudragit® RL 30 D (a 30% aqueous dispersion) was included in the experiment, the appropriate amount of the dispersion was diluted with water up to a total weight of about 300 gram. Water, or the dilute Eudragit dispersion, was then added to the dry ingredients in three portions of about 100 gram with 3 minutes of mixing after each addition. A further portion of pure water was thereafter added, followed by 1 minute of mixing, until the ingredients could form a dough. It was found that the total amount of water necessary for obtaining a dough was about 2.1 times the amount of dry ingredients (w/w).

The wet mass was transferred to a Caleva E20 extruder equipped with a 1.5 mm screen and operating at 25 rpm (revolutions per minute). The extrudate was collected on a stainless steel tray. Portions of 30-120 gram of the extrudate were then run in a Donsmark QMM-II spheronizer for up to 120 seconds at a speed of 730 rpm. The spheronized material was transferred to stainless steel trays and dried in a drying oven for 16 hours at 50°C. The dried pellets were sieved and the fraction between 1.0 and 1.4 mm was collected.

Friability testing was performed using the equipment and procedure described in European Pharmacopoeia 8.0, test 2.9.7. The pellets were sieved on a 500 μm sieve to remove any loose dust before weighing. The results are shown in table 1 below. As can be seen from entries 4-6, an increase in the amount of water (from 1.7 to 2.1 times the amount of dry ingredients (w/w)) had a positive effect on the formation of pellets.

*Experiments with different binding agents*

All experiments were performed on a 200 g scale. Pellets were manufactured at a batch size of 200 g in the extrusion step and 100 g in the spheronization step. All experiments comprised 85% w/w cholestyramine, 7.5% w/w binding agent, 4.5% w/w MCC and 3% w/w acrylate copolymer.

Cholestyramine (170 g) and microcrystalline cellulose (9 g) were charged into a planetary mixer operating at intermediate speed. Cellulose ether (15 g) was dissolved in 280 mL water. Eudragit® RL 30 D (20 g of a 30% aqueous dispersion) was added to the cellulose ether solution and the resulting liquid was slowly added to the planetary mixer in three equal portions, with mixing for 3 minutes between each addition. A final portion of pure water (between 60 and 100 g) was added to yield a wet mass of suitable texture, and mixing was performed for additionally 30 seconds. In each experiment, the total amount of liquid added was between 1.8 and 2.0 times the amount of solid material (w/w).

The wet mass was transferred to an extruder equipped with a 1.5 mm screen and operated at 25 rpm. The extrudate was collected on a stainless steel tray. Approximately 100 g of the extrudate was thereafter run in the spheronizer for 1 minute, at a speed of 730 rpm. The spheronized material was then transferred to stainless steel trays, placed in a drying oven and dried for 16 hours at 50°C. The yield was calculated as the fraction of pellets that pass through a 1.6 mm sieve but are retained on a 1.0 mm sieve.

Friability testing was performed using the equipment and procedure described in European Pharmacopoeia 8.0, test 2.9.7. The pellets were sieved on a 500 μm sieve to remove any loose dust before weighing. The results are shown in Table 2.

**Table 1**

| Entry | Amount (% w/w) | | | | Yield (%) | Friability (%) |
|---|---|---|---|---|---|---|
| | Cholestyramine | Methocel™ E3 | MCC | Eudragit® RL 30 D* | | |
| 1 | 93 | 7 | 0 | 0 | 80 | 3.2 |

(continued)

| Entry | Amount (% w/w) | | | | Yield (%) | Friability (%) |
|---|---|---|---|---|---|---|
| | Cholestyramine | Methocel™ E3 | MCC | Eudragit® RL 30 D* | | |
| 2 | 92 | 6 | 0 | 2 | 66 | 2.6 |
| 3 | 92 | 5 | 0 | 3 | 74 | 0.9 |
| 4** | 83 | 6 | 10 | 1 | 64 | 2.7 |
| 5*** | 83 | 6 | 10 | 1 | 82 | 1.1 |
| 6 | 83 | 6 | 10 | 1 | 67 | 0.5 |
| 7 | 73 | 5 | 20 | 2 | 40 | 1.5 |
| *The amount refers to dry polymer weight<br>** The total amount of water was about 1.7 times the amount of dry ingredients (w/w)<br>*** The total amount of water was about 1.9 times the amount of dry ingredients (w/w) | | | | | | |

**Table 2**

| Entry | Binding agent (cellulose ether) | Yield (%) | Friability (%) |
|---|---|---|---|
| 1 | Hydroxypropylcellulose (MW 80,000) (Klucel™ ELF Pharm) | 91 | 0.3 |
| 2 | Hydroxypropylcellulose (MW 95,000) (Klucel™ LF Pharm) | 87 | 1.3 |
| 3 | Hydroxypropyl methylcellulose (viscosity ~3 mPa·s) (Methocel™ E3 Premium LV) | 91 | 0.4 |
| 4 | Hydroxypropyl methylcellulose (viscosity ~5 mPa·s) (Methocel™ E5 Premium LV) | 92 | * |
| 5 | Methyl cellulose (Methocel™ A15 Premium LV) | 60 | 1.0 |
| 6 | Sodium carboxymethylcellulose (Blanose® NaCMC 7LF PH) | 63 | * |
| * the friability increased due to hygroscopicity. | | | |

Coating experiments (such as those in Examples 3 and 4 below) confirmed that the obtained pellets were sufficiently stable for being coated with one or more coating layers.

**Example 2**

**Disintegration testing of cholestyramine pellets**

Pellets from example 1 (10 g) are added to 400 mL of a phosphate buffer (50 mM, pH 6.8) under stirring at 300 rpm using a propeller stirrer. The time for the pellets to fully disintegrate is measured.

**Example 3**

**Formulations A-C for enzyme-controlled release**

The cholestyramine pellets of Example 1, entry 1 are formulated with a colon release coating based on Eudragit® FS 30 D and native high amylose maize starch.

The pellets composition for a unit dose comprising 250 mg cholestyramine is shown below.

| Ingredient | Amount (mg/dose) |
|---|---|
| Cholestyramine | 250 |
| Hydroxypropylcellulose (Klucel™ ELF Pharm) | 22.1 |
| Microcrystalline cellulose (Avicel® PH102) | 13.2 |
| Poly(ethyl acrylate-co-methyl methacrylate-co-trimethylammonioethyl methacrylate chloride) 1:2:0.2 (Eudragit® RL 30 D) | 8.8 |

(continued)

| Ingredient | Amount (mg/dose) |
|---|---|
| *Total* | 294.1 |

For the coating, a glycerol monostearate (GMS) emulsion containing GMS, polysorbate 80 and triethyl citrate is prepared according to general instructions from Evonik. The emulsion is then mixed with Eudragit® FS 30 D (aqueous dispersion 30%). The composition of the Eudragit FS 30 D coating dispersion, based on dry weight, is shown below. The concentration, based on dry weight, is 19.8% (w/w).

| Ingredient | Amount (w/w) |
|---|---|
| Poly(methyl acrylate-co-methyl methacrylate-co-methacrylic acid) 7:3:1 *(Eudragit® FS 30 D)* | 90.4 |
| Triethyl citrate | 4.5 |
| Glycerol monostearate 45-55 *(Kolliwax® GMS II)* | 3.6 |
| Polysorbate 80 *(Tween® 80)* | 1.5 |

The pH of the dispersion is adjusted with a 0.3 M NaOH solution to 5.5. The dispersion is mixed with a suspension of native starch granules containing 12.9% starch, 0.1% Kolliphor® SLS fine and water. The Eudragit® dispersion is mixed with the starch suspension so that the ratio between polymer film and starch in the final film is 60% starch to 40% Eudragit® FS 30 D film. The composition of the coating, based on dry weight, is shown below. The concentration, based on dry weight of the applied dispersion, is 15% (w/w).

| Ingredient | Amount (w/w) |
|---|---|
| Poly(methyl acrylate-co-methyl methacrylate-co-methacrylic acid) 7:3:1 *(Eudragit® FS 30 D)* | 36.0 |
| High amylose maize starch *(Hylon® VII)* | 59.7 |
| Triethyl citrate | 1.8 |
| Glycerol monostearate 45-55 *(Kolliwax® GMS II)* | 1.4 |
| Polysorbate 80 *(Tween® 80)* | 0.6 |
| Sodium lauryl sulphate *(Kolliphor® SLS Fine)* | 0.5 |
| NaOH | qs pH 5.5 |

The coating layer is applied using a Hüttlin Kugelcoater HKC005. The initial batch size is 75 g. The coating process is performed with an air inlet temperature of 47-52°C, resulting in a product temperature of 27-29°C. The air flow is adjusted to achieve an appropriate fluidization of the pellets during the coating.

The coating is applied to the cholestyramine pellets so as to obtain a weight gain of 84% (formulation A), 65% (formulation B) or 50% (formulation C). After the coating, the pellets are heat-treated at 40°C for 2 hours.

The coated pellets may be encapsulated in capsules, e.g. hard gelatine capsules. Details for the final formulations (on dry weight basis) are shown below:

| | Formulation A | Formulation B | Formulation C |
|---|---|---|---|
| Dose weight: | 541 mg | 485 mg | 441 mg |
| Cholestyramine: | 250 mg (46%) | 250 (52%) | 250 (57%) |
| Coating: | 247 mg (46%) | 191 (39%) | 147 (33%) |

## Example 4

### Formulations D-F for pH- and diffusion-controlled release

The cholestyramine pellets of Example 1 are formulated with a colon release coating comprising an diffusion controlled inner coating based on poly(ethyl acrylate-co-methyl methacrylate-co-trimethylammonioethyl methacrylate chloride) and an enteric outer coating based on hydroxypropyl methylcellulose acetate succinate.

Three formulations are prepared with different amounts of poly(ethyl acrylate-co-methyl methacrylate-co-trimethylammonioethyl methacrylate chloride) in the inner coating, as follows:

| | |
|---|---|
| Formulation D: | 100% Eudragit® RL 30 D |
| Formulation E: | 50% Eudragit® RL 30 D + 50% Eudragit® RS 30 D |
| Formulation F: | 100% Eudragit® RS 30 D |

The pellets composition for a unit dose comprising 250 mg cholestyramine is shown below.

| Ingredient | Amount (mg/dose) |
|---|---|
| Cholestyramine | 250 |
| Hydroxypropylcellulose *(Klucel™ ELF Pharm)* | 22.1 |
| Microcrystalline cellulose *(Avicel® PH102)* | 13.2 |
| Poly(ethyl acrylate-co-methyl methacrylate-co-trimethylammonioethyl methacrylate chloride) 1:2:0.2 *(Eudragit® RL 30 D)* | 8.8 |
| Total | 294.1 |

*Inner coating*

A glycerol monostearate (GMS) emulsion containing GMS, polysorbate 80 and triethyl citrate is prepared according to general instructions from Evonik. The emulsion is mixed with Eudragit RL30D / RS30D dispersion (30% w/w). The composition of the inner coating film, based on dry weight, is shown below. The concentration, based on dry weight of the applied dispersion, is 19.8% (w/w).

| Ingredient | Amount (w/w) |
|---|---|
| *Inner coating* | |
| Poly(ethyl acrylate-co-methyl methacrylate-co-trimethylammonioethyl methacrylate chloride) 1:2:0.2 *(Eudragit® RL 30 D)* or 1:2:0.1 *(Eudragit® RS 30 D)* | 90.4 |
| Triethyl citrate | 4.5 |
| Glycerol monostearate 45-55 *(Kolliwax® GMS II)* | 3.6 |
| Polysorbate 80 *(Tween® 80)* | 1.5 |

The coating layer is applied using a Hüttlin Kugelcoater HKC005; batch size 75 g. The coating process is performed with an air inlet temperature of 45°C, resulting in a product temperature of 27-29°C. Air flow is adjusted to achieve an appropriate fluidization of the pellets during the coating. The coating is applied to the pellets so as to obtain a weight gain of 10%. After the coating, the pellets are heat-treated at 40°C for 24 hours.

*Outer coating*

The enteric coating is prepared by mixing 7% w/w hypromellose acetate succinate, 2.45% w/w triethyl citrate, 2.1% w/w talc, 0.21% w/w sodium lauryl sulphate and 88.24% w/w water for 30 min with an overhead stirrer at low temperature, <15°C. The composition of the outer coating film, based on dry weight, is shown below. The coating liquid is kept below 15°C during the coating process.

| Ingredient | Amount (w/w) |
|---|---|
| *Outer coating* | |
| Hypromellose acetate succinate *(AQOAT AS HF)* | 59.5 |
| Triethyl citrate | 20.8 |
| Talc, micronized | 17.9 |

(continued)

| Ingredient | Amount (w/w) |
|---|---|
| *Outer coating* | |
| Sodium lauryl sulphate *(Kolliphor® SLS Fine)* | 1.8 |

The coating layer is applied using a Hüttlin Kugelcoater HKC005; batch size 75 g. The coating process is performed with an air inlet temperature of 55°C, resulting in a product temperature of 32°C. Air flow is adjusted to achieve an appropriate fluidization of the pellets during the coating. The enteric coating is applied to the pellets so as to obtain a weight gain of 40% (based on the weight of the coated pellets after application of the inner coating). After the coating, the pellets are heat-treated at 40°C/75% RH for 48 hours.

The coated pellets may be encapsulated in capsules, e.g. hard gelatine capsules. Details for the final formulations (on dry weight basis) are shown below:

| | | |
|---|---|---|
| Dose weight: | 452.9 mg | |
| Cholestyramine: | 250 mg | (55%) |
| *Inner coating:* | *29.4 mg* | |
| *Outer coating:* | *129.4 mg* | |
| Total coating: | 158.8 mg | (35%) |

### Example 5

### Sequestration assay

The sequestering capacities of the formulations is determined in a simplified assay, simulating the pH of the stomach and the small intestine. The sequestration is determined by measuring the decreasing amount of cholic acid in an aqueous solution. The USP Dissolution Apparatus 2 (paddle) Ph. Eur. 2.9.3 is used.

*Sequestration at pH 5.5*

An amount of a formulation corresponding to 250 mg cholestyramine is added to a vessel containing 500 mL of a buffered solution of cholic acid (0.192 mg/mL), pH 5.5 and the contents are stirred at 75 rpm for 6 hours. Samples of the solution are withdrawn at different time points and analysed for cholic acid by HPLC using a Thermo Hypersil Gold column, 50 mm x 2.1 mm, particle size 1.9 $\mu$m; column temperature 60 °C; mobile phase 30:70 acetonitrile: phosphate buffer (pH 3.0); flow rate 0.75 mL/min. 5 replicate samples are analysed for each formulation and the average values are calculated.

*Sequestration at pH 6.8 or 7.4*

An amount of a formulation corresponding to 250 mg cholestyramine is added to a vessel containing 250 mL 0.1 M hydrochloric acid solution (pH 1) and the contents are stirred at 75 rpm for 2 hours. 250 mL of a solution of cholic acid in potassium hydroxide/potassium phosphate buffer solution is then added to the vessel, giving a buffered solution of cholic acid (0.192 mg/mL) with pH 6.8 or 7.4. After 1 minute of mixing, a first sample is removed. The pH is thereafter verified and if necessary adjusted to 6.8 or 7.4 by addition of the appropriate amount of 0.1 M potassium hydroxide solution. The solution is thereafter mixed for an additional 6 hours. Samples of the solution are withdrawn at different time points and analysed for cholic acid by HPLC using a Thermo Hypersil Gold column, 50 mm x 2.1 mm, particle size 1.9 $\mu$m; column temperature 60 °C; mobile phase 30:70 acetonitrile: phosphate buffer (pH 3.0); flow rate 0.75 mL/min. 5 replicate samples are analysed for each formulation and the average values are calculated.

### Example 6

### *In vitro* determination of the sequestering capacity of cholestyramine formulations under simulated conditions for the gastrointestinal tract

The sequestering capacities of the cholestyramine formulations are studied in the Simulator of the Human Intestinal Microbial Ecosystem (SHIME®) as developed by ProDigest (Ghent, Belgium). The simulator is adapted to evaluate the bile acid bindingcapacity of cholestyramine formulations under physiological conditions representative for fasted stomach, small intestine and proximal colon. The liquid media representative of the fasted stomach and small intestine have previously been described by Marzorati et al. (LWT-Food Sci. Technol. 2015, vol. 60, p. 544-551). The liquid medium for the proximal colon comprises a SHIME® matrix containing a stable microbial community representative for the human colon. A method for obtaining a stable microbial community of the human intestine is described by Possemiers et al. (FEMS Microbiol. Ecol. 2004, vol. 49, p. 495-507) and references therein. The sequestration is determined by measuring the decreasing amount of bile acids in an aqueous solution. A 40:40:20 (w/w) mixture of cholic acid (CA), chenodeoxycholic acid (CDCA) and deoxycholic acid (DCA) is used as a representative mixture of human bile salts (Carulli et al., Aliment. Pharmacol. Ther. 2000, vol. 14, issue supplement s2, p. 14-18).

A comparative experiment to which pure (unformulated) cholestyramine powder is added is also conducted. A control experiment to which no cholestyramine is added is conducted in order to monitor the degradation of the bile salts under the colonic conditions used in the assay.

Each experiment is performed in triplicate to account for biological variation.

*Fasted stomach*

Amounts of formulations A, B and C corresponding to 91 mg of cholestyramine and the pure cholestyramine (91 mg) are dosed to 14 mL fasted stomach liquid medium (pH 1.8). The digests are incubated for 1 hour at 37 °C.

*Small intestine*

After one hour of stomach incubation, 5.6 mL pancreatic juice (pH 6.8) containing the defined 40:40:20 mixture of bile salts (46.7 mM) is added. The small intestine digests are incubated for 2 hours at 37 °C and samples are taken after 0, 60 and 120 minutes.

*Proximal colon*

After two hours of small intestine incubation, 42 mL of a full SHIME® matrix (pH 6.0) originated from the ascending colon of a SHIME® system is added. The colon digests are incubated for 24 hours at 37 °C and samples are collected every hour for the first 6 hours and then at 19h and at 24h.

*Sample analysis*

The concentration of free bile salts in the samples is assessed by means of HPLC. A calibration curve is used to calculate the concentrations of CA, CDCA and DCA in the samples. One mL of each sample is centrifuged for 2 min at 5000 g. 500 $\mu$L of the supernatant is mixed with 500 $\mu$L of an 80:20 (v:v) mixture of methanol and phosphate buffer, vigorously vortexed, filtered through a 0.2 $\mu$m PTFE filter and injected in a Hitachi Chromaster HPLC equipped with a UV-Vis detector. The three bile salts are separated by a reversed-phase C18 column (Hydro-RP, 4 $\mu$m, 80 Å, 250 x 4.6 mm, Synergi). The separation is performed under isocratic conditions at room temperature, using a 80:20 (v:v) mixture of methanol and phosphate buffer as the mobile phase. The analysis is performed at 0.7 mL/min during 23 minutes and the bile salts are detected at 210 nm. The injection volume is set at 20 $\mu$L for stomach and small intestine samples and 50 $\mu$L for colon samples.

The full SHIME® matrix that is used for the colonic incubations contains (degraded) bile salts originating from BD Difco™ Oxgall, a dehydrated fresh bile extract from bovine origin (Catalog Number 212820). Although the exact composition of this mixture is unknown, a higher quantity of free bile salts might be expected in the colon samples. The values of the background (i.e. blank sample where no mix of bile salts is added) are therefore subtracted from each sample in order to take into account the 'baseline' of free bile salts present in the total SHIME® matrix.

The measured concentrations of the different bile acids in the control sample will show the effect and extent of microbial salt metabolism in the gut (e.g. deconjugation, dehydrogenation and dehydroxylation), particularly in the colon. A sudden and large decrease of the concentrations of CA, CDCA and DCA in the control sample may be observed during the transition of the small intestinal to the colonic incubation.

The percentage of remaining bile acids in each studied sample may be calculated as the ratio of the value of the studied sample to the value of the control sample at the corresponding incubation time. A plot of the percentage of remaining bile acids against time will show the decrease of bile acids, i.e. the sequestration of bile acids by the cholestyramine formulations, during small intestinal and colonic incubation.

## EP 3 664 782 B1

**Claims**

1.  A population of extruded and spheronized pellets, each extruded and spheronized pellet comprising at least 70% w/w cholestyramine and

    i. a combination of at least 6% w/w of a cellulose ether and at least 2% w/w of an acrylate copolymer; or
    ii. a combination of at least 5% w/w of a cellulose ether and at least 3% w/w of an acrylate copolymer; or
    iii. a combination of at least 6% w/w of a cellulose ether, at least 1% w/w of an acrylate copolymer and at least 10% w/w microcrystalline cellulose; or
    iv. a combination of at least 5% w/w of a cellulose ether, at least 2% w/w of an acrylate copolymer and at least 20% w/w microcrystalline cellulose.

2.  The pellets according to claim 1, wherein the cellulose ether is methyl cellulose, hydroxypropylcellulose, hydroxypropyl methylcellulose or sodium carboxymethyl cellulose, or a mixture comprising two or more of these cellulose ethers.

3.  The pellets according to claim 1 or 2, wherein the acrylate copolymer is an ammonio methacrylate copolymer.

4.  The pellets according to any one of claims 1 to 3, wherein the pellets comprise at least 85% w/w cholestyramine and wherein the pellets are according to the alternatives (i) or (ii) of claim 1.

5.  The pellets according to any one of claims 1 to 4, wherein the diameter of the pellets is from 1000 $\mu$m to 1600 $\mu$m.

6.  The pellets according to any one of claims 1 to 5, formulated for colon targeted delivery.

7.  A multiparticulate drug delivery system comprising a plurality of cholestyramine pellets according to any one of claims 1 to 6.

8.  The drug delivery system according to claim 7, wherein the cholestyramine pellets are formulated for colon targeted delivery.

9.  The drug delivery system according to claim 8, wherein the colon targeted delivery is based on an enzyme-controlled release or on a pH- and diffusion-controlled release.

10. An oral formulation for targeted delivery of cholestyramine to the colon, comprising

    a) a plurality of pellets according to any one of claims 1 to 5; and
    b) a colon release coating around said pellets.

11. The formulation according to claim 10, wherein the colon release coating comprises starch, preferably resistant starch type 2 (RS2).

12. The formulation according to claim 10, wherein the colon release coating comprises a diffusion-controlled inner coating layer and an enteric outer coating layer.

13. The formulation according to claim 12, wherein the diffusion-controlled inner coating layer comprises poly(ethyl acrylate-co-methyl methacrylate-co-trimethylammonioethyl methacrylate chloride) 1:2:0.2, 1:2:0.1 or a combination thereof.

14. The formulation according to claim 12 or 13, wherein the enteric outer coating layer comprises hydroxypropyl methylcellulose acetate succinate.

15. The formulation according to any one of claims 10 to 14, for use in the treatment or prevention of bile acid malabsorption.

16. The formulation for use according to claim 15, wherein the bile acid malabsorption is the result of ileal disease (Crohn's disease), ileal resection or ileal bypass, the result of overproduction of bile acids or defective feedback inhibition of hepatic bile acid synthesis, or the result of cholecystectomy, vagotomy, small intestinal bacterial overgrowth (SIBO), coeliac disease, pancreatic insufficiency (chronic pancreatitis, cystic fibrosis), pancreatic transplant, radiation enteritis, collagenous colitis, microscopic colitis, lymphocytic colitis, ulcerative colitis or irritable bowel syndrome (IBS-D).

17. The formulation according to any one of claims 10 to 14, for use in the treatment or prevention of bile acid diarrhoea, such as upon oral administration of an IBAT inhibitor.

18. The formulation for use according to claim 17, wherein the IBAT inhibitor is

1,1-dioxo-3,3-dibutyl-5-phenyl-7-methylthio-8-(N-{(R)-α-[N-((S)-1-carboxypropyl)-carbamoyl]-4-hydroxybenzyl}carbamoylmethoxy)-2,3,4,5-tetrahydro-1,2,5-benzothiadiazepine; or
1,1-dioxo-3,3-dibutyl-5-phenyl-7-methylthio-8-(N-{(R)-1'-phenyl-1'-[N'-(carboxymethyl)-carbamoyl]methyl}carbamoylmethoxy)-2,3,4,5-tetrahydro-1,5-benzothiazepine;

or a pharmaceutically acceptable salt thereof.

**Patentansprüche**

1. Eine Population von extrudierten und sphäronisierten Pellets, wobei jedes extrudierte und sphäronisierte Pellet mindestens 70 Gew.-% Cholestyramin und

i. eine Kombination aus mindestens 6 Gew.-% eines Celluloseethers und mindestens 2 Gew.-% eines Acrylatcopolymers; oder
ii. einer Kombination aus mindestens 5 Gew.-% eines Celluloseethers und mindestens 3 Gew.-% eines Acrylatcopolymers; oder
iii. eine Kombination aus mindestens 6 Gew.-% eines Celluloseethers, mindestens 1 Gew.-% eines Acrylatcopolymers und mindestens 10 Gew.-% mikrokristalliner Cellulose; oder
iv. eine Kombination aus mindestens 5 Gew.-% eines Celluloseethers, mindestens 2 Gew.-% eines Acrylatcopolymers und mindestens 20 Gew.-% mikrokristalliner Cellulose umfasst.

2. Pellets nach Anspruch 1, wobei der Celluloseether Methylcellulose, Hydroxypropylcellulose, Hydroxypropylmethylcellulose oder Natriumcarboxymethylcellulose oder eine Mischung umfasssend zwei oder mehr dieser Celluloseether ist.

3. Pellets nach Anspruch 1 oder 2, wobei das Acrylatcopolymer ein Ammoniummethacrylatcopolymer ist.

4. Pellets nach einem der Ansprüche 1 bis 3, wobei die Pellets mindestens 85 Gew.-% Cholestyramin umfassen und wobei die Pellets den Alternativen (i) oder (ii) von Anspruch 1 entsprechen.

5. Pellets nach einem der Ansprüche 1 bis 4, wobei der Durchmesser der Pellets 1000 μm bis 1600 μm beträgt.

6. Pellets nach einem der Ansprüche 1 bis 5, formuliert für eine auf den Dickdarm gezielte Abgabe.

7. Multipartikuläres Arzneimittelabgabesystem, umfassend eine Vielzahl von Cholestyraminpellets nach einem der Ansprüche 1 bis 6.

8. Arzneimittelabgabesystem nach Anspruch 7, wobei die Cholestyraminpellets für eine auf den Dickdarm gezielte Abgabe formuliert sind.

9. Arzneimittelabgabesystem nach Anspruch 8, wobei die auf den Dickdarm gerichtete Abgabe auf einer enzymgesteuerten Freisetzung oder auf einer pH- und diffusionsgesteuerten Freisetzung basiert.

10. Orale Formulierung zur gezielten Abgabe von Cholestyramin an den Dickdarm, umfassend

a) eine Vielzahl von Pellets nach einem der Ansprüche 1 bis 5; und
b) eine Beschichtung zur Freisetzung im Dickdarm um die Pellets herum.

**11.** Formulierung nach Anspruch 10, wobei die Beschichtung zur Freisetzung im Dickdarm Stärke, vorzugsweise resistente Stärke Typ 2 (RS2), umfasst.

**12.** Formulierung nach Anspruch 10, wobei die Beschichtung zur Freisetzung im Dickdarm eine diffusionsgesteuerte innere Überzugsschicht und eine enterische äußere Überzugsschicht umfasst.

**13.** Formulierung nach Anspruch 12, wobei die diffusionsgesteuerte innere Überzugsschicht Poly(ethylacrylat-co-methylmethacrylat-co-trimethylammonioethylmethacrylatchlorid) 1:2:0,2, 1:2:0,1 oder eine Kombination davon umfasst.

**14.** Formulierung nach Anspruch 12 oder 13, wobei die enterische äußere Überzugsschicht Hydroxypropylmethylcelluloseacetatsuccinat umfasst.

**15.** Formulierung nach einem der Ansprüche 10 bis 14 zur Verwendung bei der Behandlung oder Vorbeugung von Gallensäure-Malabsorption.

**16.** Formulierung zur Verwendung nach Anspruch 15, wobei die Gallensäure-Malabsorption das Ergebnis einer ilealen Erkrankung (Morbus Crohn), einer ilealen Resektion oder eines ilealen Bypasses, das Ergebnis einer Überproduktion von Gallensäure oder einer fehlerhaften Feedback-Hemmung der hepatischen Gallensäuresynthese oder das Ergebnis einer Cholezystektomie, Vagotomie, bakteriellen Überwucherung des Dünndarms (SIBO), Zöliakie, Pankreasinsuffizienz (chronische Pankreatitis, zystische Fibrose), Pankreastransplantation, Strahlenenteritis, kollagener Kolitis, mikroskopischer Kolitis, lymphozytärer Kolitis, Colitis ulcerosa oder Reizdarmsyndrom (IBS-D) ist.

**17.** Formulierung nach einem der Ansprüche 10 bis 14 zur Verwendung bei der Behandlung oder Vorbeugung von Gallensäurediarrhö, wie z B. bei oraler Verabreichung eines IBAT-Inhibitors.

**18.** Formulierung zur Verwendung nach Anspruch 17, wobei der IBAT-Inhibitor 1,1-Dioxo-3,3-dibutyl-5-phenyl-7-methylthio-8-(N-{(R)-α-[N- ((S)-1-Carboxypropyl)-carbamoyl]-4-hydroxybenzyl}carbamoylmethoxy)-2,3,4,5-tetrahydro-1,2,5-benzothiadiazepin ist; oder 1,1-Dioxo-3,3-dibutyl-5-phenyl-7-methylthio-8-(N-{(R)-1'-phenyl-1'-[N'-(carboxymethyl)-carbamoyl]methyl}carbamoylmethoxy)-2,3,4,5-tetrahydro-1,5-benzothiazepin; oder ein pharmazeutisch verträgliches Salz davon.

**Revendications**

**1.** Population de pastilles extrudées et sphéronisées, chaque pastille extrudée et sphéronisée comprenant au moins 70 % p/p de cholestyramine et

    i. une combinaison d'au moins 6 % p/p d'un éther de cellulose et d'au moins 2 % p/p d'un copolymère d'acrylate ; ou
    ii. une combinaison d'au moins 5 % p/p d'un éther de cellulose et d'au moins 3 % p/p d'un copolymère d'acrylate ; ou
    iii. une combinaison d'au moins 6 % p/p d'un éther de cellulose, d'au moins 1 % p/p d'un copolymère d'acrylate et d'au moins 10 % p/p de cellulose microcristalline ; ou
    iv. une combinaison d'au moins 5 % p/p d'un éther de cellulose, d'au moins 2 % p/p d'un copolymère d'acrylate et d'au moins 20 % p/p de cellulose microcristalline.

**2.** Pastilles selon la revendication 1, dans lesquelles l'éther de cellulose est la méthylcellulose, l'hydroxypropylcellulose, l'hydroxypropylméthylcellulose ou la carboxyméthylcellulose de sodium, ou un mélange comprenant deux ou plusieurs de ces éthers de cellulose.

**3.** Pastilles selon la revendication 1 ou 2, dans lesquelles le copolymère d'acrylate est un copolymère d'ammonio-méthacrylate.

**4.** Pastilles selon l'une quelconque des revendications 1 à 3, dans lesquelles les pastilles comprennent au moins 85 % p/p de cholestyramine et dans lesquelles les pastilles sont selon les variantes (i) ou (ii) de la revendication 1.

**5.** Pastilles selon l'une quelconque des revendications 1 à 4, dans lesquelles le diamètre des pastilles est de 1000 $\mu$m à

1600 μm.

**6.** Pastilles selon l'une quelconque des revendications 1 à 5, formulées pour une administration ciblée sur le côlon.

**7.** Système d'administration de médicament multiparticulaire comprenant une pluralité de pastilles de cholestyramine selon l'une quelconque des revendications 1 à 6.

**8.** Système d'administration de médicament selon la revendication 7, dans lequel les pastilles de cholestyramine sont formulées pour une administration ciblée sur le côlon.

**9.** Système d'administration de médicament selon la revendication 8, dans lequel l'administration ciblée sur le côlon est basée sur une libération contrôlée par enzyme ou sur une libération contrôlée par pH et diffusion.

**10.** Formulation orale pour une administration ciblée de cholestyramine dans le côlon, comprenant

a) une pluralité de pastilles selon l'une quelconque des revendications 1 à 5 ; et
b) un revêtement de libération du côlon autour desdites pastilles.

**11.** Formulation selon la revendication 10, dans laquelle le revêtement de libération du côlon comprend de l'amidon, de préférence de l'amidon résistant de type 2 (RS2).

**12.** Formulation selon la revendication 10, dans laquelle le revêtement de libération du côlon comprend une couche de revêtement interne contrôlée par diffusion et une couche de revêtement externe entérique.

**13.** Formulation selon la revendication 12, dans laquelle la couche de revêtement interne contrôlée par diffusion comprend du chlorure de poly(acrylate d'éthyle-cométhacrylate de méthyle-co-méthacrylate de triméthylammonioéthyle) 1:2:0,2, 1:2:0,1 ou une combinaison de ceux-ci.

**14.** Formulation selon la revendication 12 ou 13, dans laquelle la couche de revêtement externe entérique comprend du succinate d'acétate d'hydroxypropylméthylcellulose.

**15.** Formulation selon l'une quelconque des revendications 10 à 14, destinée à être utilisée dans le traitement ou la prévention de malabsorption des acides biliaires.

**16.** Formulation destinée à être utilisée selon la revendication 15, dans laquelle la malabsorption des acides biliaires est le résultat d'une maladie iléale (maladie de Crohn), d'une résection iléale ou d'un pontage iléal, le résultat d'une surproduction d'acides biliaires ou d'une rétro-inhibition défectueuse de la synthèse des acides biliaires hépatiques, ou le résultat d'une cholécystectomie, d'une vagotomie, d'une prolifération bactérienne de l'intestin grêle (SIBO), d'une maladie cœliaque, d'une insuffisance pancréatique (pancréatite chronique, fibrose kystique), d'une transplantation pancréatique, d'une entérite par rayonnement, d'une colite collagène, d'une colite microscopique, d'une colite lymphocytaire, d'une colite ulcéreuse ou d'un syndrome du côlon irritable (IBS-D).

**17.** Formulation selon l'une quelconque des revendications 10 à 14, destinée à être utilisée dans le traitement ou la prévention de la diarrhée due aux acides biliaires, par exemple lors de l'administration orale d'un inhibiteur de l'IBAT.

**18.** Formulation destinée à être utilisée selon la revendication 17, dans laquelle l'inhibiteur de l'IBAT est la 1,1-dioxo-3,3-dibutyl-5-phényl-7-méthylthio-8-  (*N*-{(R)-α-[*N*-((S)-1-carboxypropyl)-carbamoyl]-4-hydroxybenzyl}carbamoylméthoxy)-2,3,4,5-tétrahydro-1,2,5-benzothiadiazépine ; ou 1,1-dioxo-3,3-dibutyl-5-phényl-7-méthylthio-8-(*N*-{(R)-1'-phényl-1'-[N'-(carboxyméthyl)-carbamoyl]méthyl}carbamoylméthoxy)  -2,3,4,5-tétrahydro-1,5-benzothiazépine  ; ou un sel pharmaceutiquement acceptable de celui-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1273307 A
- WO 2017138876 A
- WO 2017138877 A
- WO 2017138878 A
- US 5167965 A
- WO 9316055 A
- WO 9418183 A
- WO 9418184 A
- WO 9605188 A
- WO 9608484 A
- WO 9616051 A
- WO 9733882 A
- WO 9803818 A
- WO 9807449 A
- WO 9840375 A
- WO 9935135 A
- WO 9964409 A
- WO 9964410 A
- WO 0047568 A
- WO 0061568 A
- WO 0038725 A
- WO 0038726 A
- WO 0038727 A
- WO 0038728 A
- WO 0038729 A
- WO 0168096 A
- WO 0232428 A
- WO 03061663 A
- WO 2004006899 A
- WO 2007009655 A
- WO 2007009656 A
- DE 19825804
- EP 864582 A
- EP 489423 A
- EP 549967 A
- EP 573848 A
- EP 624593 A
- EP 624594 A
- EP 624595 A
- EP 624596 A
- EP 0864582 A
- EP 1173205 A
- EP 1535913 A
- WO 0166533 A
- WO 0250051 A
- WO 03022286 A
- WO 03020710 A
- WO 03022825 A
- WO 03022830 A
- WO 03091232 A
- WO 03106482 A
- WO 2004076430 A
- WO 9932478 A
- WO 0001687 A
- WO 0168637 A
- WO 03022804 A
- WO 2008058628 A
- WO 2008058630 A

**Non-patent literature cited in the description**

- **PATTNI** ; **WALTERS**. *Br. Med. Bull.*, 2009, vol. 92, 79-93
- **ISLAM** ; **DI BAISE**. *Pract. Gastroenterol.*, 2012, vol. 36 (10), 32-44
- *CHEMICAL ABSTRACTS*, 11041-12-6
- **JACOBSEN et al.** *Br. Med. J.*, 1985, vol. 290, 1315-1318
- **VERTOMMEN** ; **KINGET**. *Drug Dev. Ind. Pharm.*, 1997, vol. 23, 39-46
- **SINHA** ; **KUMRIA**. *Eur. J. Pharm. Sci.*, 2003, vol. 18, 3-18
- **MARZORATI et al.** *LWT-Food Sci. Technol.*, 2015, vol. 60, 544-551
- **POSSEMIERS et al.** *FEMS Microbiol. Ecol.*, 2004, vol. 49, 495-507
- **CARULLI et al.** *Aliment. Pharmacol. Ther.*, 2000, vol. 14 (s2), 14-18